(19) Europäisches Patentamt
European Patent Office
Office européen des brevets

(11) Veröffentlichungsnummer: **0 185 220**
**A2**

(12) # EUROPÄISCHE PATENTANMELDUNG

(21) Anmeldenummer: **85114898.1**

(22) Anmeldetag: **25.11.85**

(51) Int. Cl.4: **C07D 501/36**

(30) Priorität: **19.12.84 CH 6009/84**

(43) Veröffentlichungstag der Anmeldung:
**25.06.86 Patentblatt 86/26**

(84) Benannte Vertragsstaaten:
**AT BE CH DE FR GB IT LI NL**

(71) Anmelder: **F. HOFFMANN-LA ROCHE & CO. Aktiengesellschaft**
**CH-4002 Basel(CH)**

(72) Erfinder: **Furlenmeier, André, Dr.**
**Wettsteinallee 119**
**CH-4058 Basel(CH)**

(74) Vertreter: **Notegen, Eric-André et al**
**Grenzacherstrasse 124 Postfach 3255**
**CH-4002 Basel(CH)**

(54) **Zwischenprodukte zur Herstellung von Cephalosporinen.**

(57) Die neuen Verbindungen der Formel

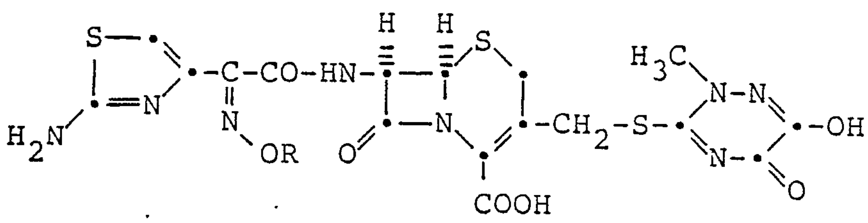

II

worin R niederes Alkanoyl bedeutet,

können durch Behandeln mit einem Gemisch aus Wasser und einem primären $C_{1-3}$-Alkohol und einer anorganischen Base bei einem pH von 6-9 in das antibiotisch wirksame Cephalosporin 7-[2-(2-Amino-4-thiazolyl)-2-syn-hydroxyiminoacetamido]-3-{[(2,5-dihydro-6-hydroxy-2-methyl-5-oxo-as-3-triazinyl)thio]methyl}-3-cephem-4-carbonsäure übergeführt werden.

EP 0 185 220 A2

Zwischenprodukte zur Herstellung von Cephalosporinen

Die vorliegende Erfindung betrifft neue Zwischenprodukte zur Herstellung von 7-[2-(2-Amino-4-thiazolyl)-2-syn-hydroxyiminoacetamido] - 3-{[(2,5-dihydro-6-hydroxy-2-methyl-5-oxo-as - 3-triazinyl)thio]methyl}-3-cephem-4-carbonsäure der Formel

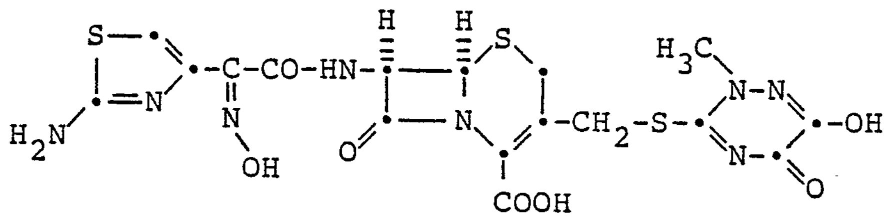

I

einem bekannten Cephalosporin mit wertvollen antibiotischen, insbesondere antibakteriellen Eigenschaften, und deren pharmazeutisch annehmbaren Salzen.

Bei den neuen Zwischenprodukten handelt es sich um Verbindungen der Allgemeinen Formel

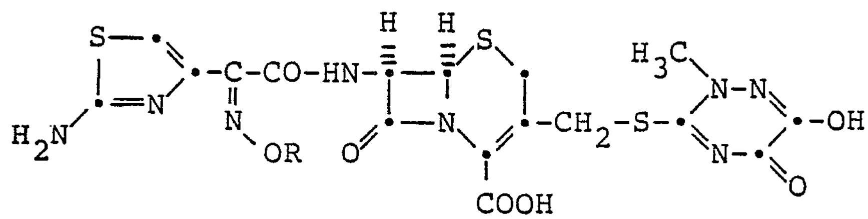

II

worin R niederes Alkanoyl bedeutet,

und um deren Salze mit Basen.

Gegenstand der vorliegenden Erfindung sind: Die vorerwähnten Verbindungen der Formel II und deren Salze mit Basen, ein Verfahren zu deren Herstellung und deren Verwendung zur Herstellung der Verbindung der obigen Formel I und deren pharmazeutisch annehmbaren Salzen.

Der Ausdruck "nieder" bezeichnet Reste und Verbindungen mit höchstens 7, vorzugsweise höchstens 4 Kohlenstoffatomen. Der Ausdruck "Alkanoyl" bezeichnet geradkettige oder verzweigte Fettsäurereste, wie Acetyl, Propanoyl und Isobutyryl.

In einer bevorzugten Ausführungsform betrifft die vorliegende Erfindung die Verbindung der Formel II, worin R Acetyl bedeutet, d.h. die 7-[2-(2-Amino-4-thiazolyl)-2-syn-acetoxyiminoacetamido] - 3-{[(2,5-dihydro-6-hydroxy-2 - methyl-5-oxo-as-3-triazinyl)thio]methyl}-3-cephem-4-carbonsäure, und deren Salze mit Basen.

Die Verbindungen der Formel II und deren Salze mit Basen können erfindungsgemäss dadurch hergestellt werden, dass man die Verbindung der Formel

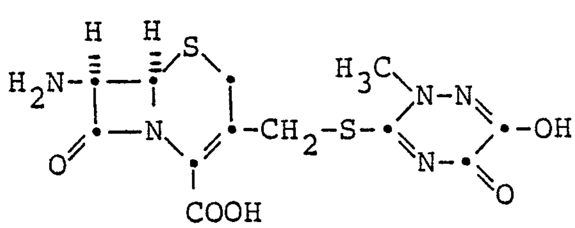

mit einer Verbindung der allgemeinen Formel

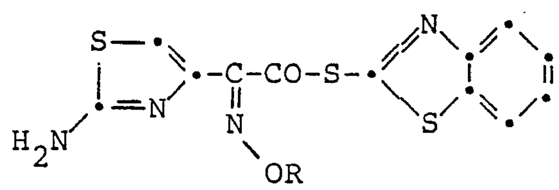

IV

worin R niederes Alkanoyl bedeutet,

acyliert und die erhaltene Verbindung der Formel II erwünschtenfalls mit einer Base in ein Salz überführt.

Geeignete Lösungsmittel für die Umsetzung der Verbindung der Formel III mit einer Verbindung der Formel IV sind beispielsweise N,N-Diemthylformamid, Diemthylsulfoxid, Aether, wie Tetrahydrofuran und Dioxan, und Mischungen dieser Lösungsmittel mit Wasser. Die Umsetzung wird vorzugsweise in Gegenwart eines tertiären Amins, wie Triäthylamin, N-Methylpyrrolidin und N-Methylmorpholin, und in einem Temperaturbereich von etwa 0°C bis Raumtemperatur durchgeführt.

Die Verbindungen der Formel II können erwünschtenfalls mit Basen in Salzen übergeführt werden. Es kommen dabei sowohl Salze mit anorganischen als auch Salze mit organischen Basen in Betracht, beispielsweise Alkalimetallsalze, wie die Natrium- und Kaliumsalze, und Ammoniumsalze und Salze mit Aminen, wie die Triäthylammonium- und Pyridiniumsalze. Derartige Salze können nach an sich bekannten und jedem Fachmann geläufigen Methoden hergestellt werden.

Die als Ausgangsstoffe verwendeten Verbindungen der Formel IV können beispielsweise gemäss dem nachfolgenden Formelschema I hergestellt werden, worin R' niederes Alkyl, $M^+$ ein Alkalimetall-Kation und $Y^+$ ein quaternäres N-Allyl-ammoniumion bedeuten, und R obige Bedeutung besitzt:

Formelschema I

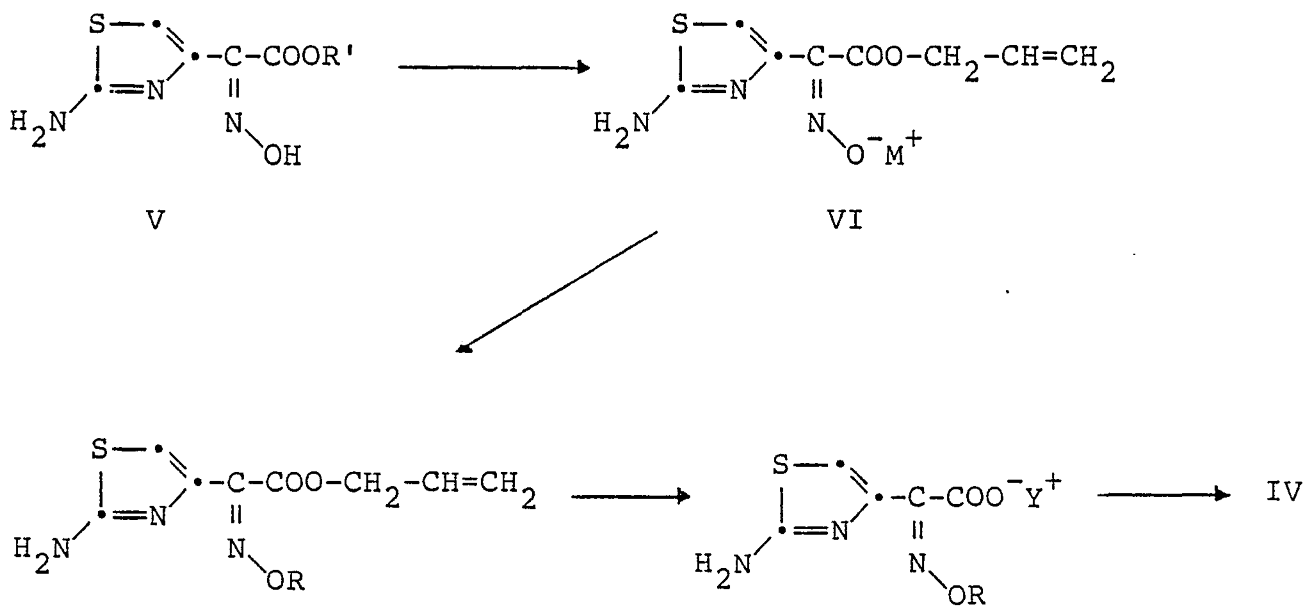

V

VI

IV

VII

VIII

Die Herstellung einer Verbindung der Formel VI aus einer Verbindung der Formel V erfolgt vorzugsweise durch Umsetzen der letzteren mit einem Alkalimetallallylat, wie Kaliumallylat, wobei man als Lösungsmittel vorzugsweise Allylalkohol verwendet. Die Reaktion erfolgt vorzugsweise bei Raumtemperatur. Die mit R' bezeichnete niedere Alkylgruppe in einer Verbindung der Formel V kann geradkettig oder verzweigt sein. R' kann beispielsweise Methyl, Aethyl, Propyl oder 2-Butyl bedeuten, wobei die Bedeutungsmöglichkeit Aethyl bevorzugt ist.

Durch Alkanoylierung einer Verbindung der Formel VI in einem geeigneten Lösungsmittel erhält man eine Verbindung der Formel VII. Geeignete Lösungsmittel sind beispielsweise Aether, wie Tetrahydrofuran und Dioxan, N,N-Dimethylformamid und Dimethylsulfoxid. Geeignete Alkanoylierungsmittel sind beispielsweise die entsprechenden Säurehalogenide, wie Acetylchlorid, die entsprechenden Säureimidazolide, wie das Essigsäureimidazolid. Die Reaktion wird vorzugsweise in einem Temperaturbereich von etwa 0°C bis etwa Raumtemperatur durchgeführt.

Durch Behandeln einer Verbindung der Formel VII mit einem tertiären Amin in Gegenwart einer Palladiumverbindung und eines Tri(niederen alkyl)phosphites, wie Triäthylphosphit, erhält man eine Verbindung der Formel VIII. Geeignete Palladiumverbindungen sind beispielsweise Palladium(II)salze mit Halogenwasserstoffsäuren, wie Chlor- oder Bromwasserstoffsäure, oder niederen Fettsäuren, wie Essigsäure oder Propionsäure. Geeignete tertiäre Amine sind beispielsweise Tri(niedere alkyl)amine, wie Trimethylamin und Triäthylamin, N-(niedere Alkyl)pyrrolidine, wie N-Methylpyrrolidin, N-(niedere Alkyl)morpholine, wie N-Methylmorpholin und dergleichen. Als Lösungsmittel kommen beispielsweise Acetonitril, halogenierte Kohlenwasserstoffe, wie Methylenchlorid, und Essigester in Frage. Die Reaktion wird vorzugsweise in einem Temperaturbereich von etwa 0°C bis etwa Raumtemperatur durchgeführt. In der erhaltenen Verbindung der Formel VIII bedeutet das Symbol $Y^+$ das N-Allylammoniumion des eingesetzten tertiären Amins.

Durch Umsetzen einer Verbindung der Formel VIII mit 2,2-Dithio-bis-benzothiazol in Gegenwart eines tertiären Amins und eines Tri(niederen alkyl)phosphites in einem geeigneten Lösungsmittel erhält man eine Verbindung der Formel IV. Geeignete tertiäre Amine sind beispielsweise die im Zusammenhang mit der Herstellung der Verbindungen der Formel VIII erwähnten tertiären Amine. Als Tri(niederes alkyl) phosphit verwendet man vorzugsweise Triäthylphosphit. Geeignete Lösungsmittel sind beispielsweise Acetonitril, halogenierte Kohlenwasserstoffe, wie Methylenchlorid, und Essigester. Die Reaktion wird vorzugsweise in einem Temperaturbereich von etwa 0°C bis etwa Raumtemperatur durchgeführt.

Wie bereits erwähnt können die Verbindungen der Formel II und Salze davon mit Basen zur Herstellung der Verbindung der Formel I und deren pharmazeutisch annehmbaren Salzen verwendet werden. In einer speziellen Ausführungsform können die Verbindung der Formel I und deren pharmazeutisch annehmbare Salze dadurch hergestellt werden, dass man eine Verbindung der Formel II oder ein Salz davon mit einer Base bei einem pH von 6-9 mit einem Gemisch aus Wasser und einem primären $C_{1-3}$-Alkohol und einer anorganischen Base behandelt und die erhaltene Verbindung der Formel I erwünschtenfalls in ein pharmazeutisch annehmbares Salz überführt.

Der Ausdruck "primärer $C_{1-3}$-Alkohol" bezeichnet die Alkohole Methanol, Aethanol und Propanol. Ein für den vorliegenden Zweck besonders geeigneter Alkohol ist das Methanol. Bevorzugte anorganische Basen sind die Alkalimetallhydroxide, carbonate und -bicarbonate, wie Natriumhydroxid, Kaliumhydroxid, Natriumcarbonat, Kaliumcarbonat, Natriumbicarbonat und Kaliumbicarbonat. Diese anorganischen Basen werden zweckmässigerweise in Form von wässrigen Lösungen, beispielsweise in Form von 1 bis 2N-Lösungen, verwendet. In einer bevorzugten Ausführungsform verwendet man Natronlauge als anorganische Base.

Die reaktion wird vorzugsweise in einem pH-Bereich von 7,5-8,5 durchgeführt. Zweckmässigerweise sorgt man dafür, dass der pH während der ganzen Reaktionsdauer konstant bleibt. Zu diesem Zweck kann man beispielsweise einen Autotitrator verwenden. Das es sich bei der obigen Reaktion nicht um eine Hydrolyse, sondern um eine Umesterung handelt, ist der gesamte Verbrauch an anorganischer Base nur sehr gering.

Das Verhältnis von Wasser zu primärem $C_{1-3}$-Alkohol ist nicht besonders kritisch und kann in einem breiten Rahmen variieren. Vorzugsweise verwendet man ein Verhältnis von Wasser zu primärem $C_{1-3}$ -Alkohol von 1:5 bis 5:1, wobei ein Verhältnis von 1:1 bis 1:2 besonders bevorzugt wird.

Die Verbindung der Formel I kann erwünschtenfalls mit Säuren oder Basen in pharmazeutisch annehmbare Salze übergeführt werden, wobei deren Herstellung nach an sich bekannten und jedem Fachmann geläufigen Methoden erfolgen kann. Es kommen dabei sowohl Salze mit anorganischen als auch Salze mit organischen Säuren und Basen in Betracht.

Geeignete pharmazeutisch annehmbare Säureadditionssalze sind beispielsweise Hydrochloride, Hydrobromide, Sulfate, Nitrate, Citrate, Acetate, Maleate, Succinate, Methansulfonate, p-Toluolsulfonate und dergleichen.

Geeignete pharmazeutisch annehmbare Salze mit Basen sind beispielsweise die Alkalimetallsalze, wie die Natrium- und die Kaliumsalze, Erdalkalimetallsalze, Ammoniumsalze, Salze mit Aminen, wie Triäthylamin, N-Methylglucamin, Diäthanolamin und Triäthanolamin, Salze mit basischen Aminosäuren, wie Arginin, Ornitin, Lysin und Hystidin, und dergleichen.

Das nachfolgende Beispiel dient der näheren Erläuterung der vorliegenden Erfindung, soll jedoch deren Umfang in keiner Weise beschränken.

Beispiel

a) Man löst 33,7 g (600 mM) Kaliumhydroxid in 200 ml Allylalkohol, gibt 300 ml Toluol dazu, filtriert die leicht trübe Lösung und engt bei 30°C im Vakuum ein (Entfernung von Wasser). Der ölige Rückstand wird nochmals in 300 ml Toluol aufgenommen und die Lösung wird erneut im Vakuum eingeengt. Das erhaltene Kaliumallylat wird in 900 ml Allylalkohol gelöst, worauf man mit 64,5 g (300 mM) 2-(2-Amino-4-thiazolyl)-2-syn-hydroxyimino-essigsäure-äthylester versetzt und 2 Tage bei Raumtemperatur rührt. Das auskristallisierte Material wird abgenutscht, sukzessive mit Allylalkohol, Essigester und Aether gewaschen und im Vakuum bei 40°C getrocknet. Man erhält das Kaliumsalz des 2-(2-Amino-4-thiasolyl)-2-syn-hydroxyimino-essigsäure-allylesters vom Smp. > 250°C.

b) Man suspendiert 76,2 g (287,2 mM) des Kaliumsalzes des 2-(2-Amino-4-thiazolyl) - 2-syn-hydroxyimino-essigsäure-allylesters in 1,4 l Tetrahydrofuran, kühlt auf 0°C ab und versetzt unter Rühren mit 20,4 ml (287,2 mM) Acetylchlorid. Nach 1 Stunde wird auf ein kleines Volumen eingeengt und mit 3 l Essigester versetzt. Die erhaltene Lösung wird dreimal mit Wasser gewaschen, und die wässrigen Extrakte werden mit 1 l Essigester ausgeschüttelt. Die dunkel gefärbte organische Phase wird mit Tierkohle behandelt, über Magnesiumsulfat getrocknet und im Vakuum eingedampft. Man nimmt den Rückstand in 1 l Methylenchlorid auf, filtriert, um ungelöstes Material zu entfernen, versetzt dann mit 2 l Tetrachlorkohlenstoff und engt auf ein kleines Volumen ein. Das auskristallisierte Material wird abgenutscht, mit Tetrachlorkohlenstoff und Petroläther gewaschen und im Vakuum bei 40°C getrocknet. Man erhält 2-(2-Amino-4-thiazolyl) - 2-syn-acetoxyimino-essigsäure-allylester vom Smp. 144-147°C.

c) Man löst 54 g (200 mM) 2-(2-Amino-4-thiazolyl) -

2-syn-acetoxyimino-essigsäure-allylester unter Stickstoff in 1,4 l Acetonitril, kühlt auf 0°C und versetzt sukzessive mit 448 mg (2 mM) Palladium(II)acetat und 1,72 ml (10 mM) Triäthylphosphit. Nach 5 Minuten gibt man 23,2ml (220 mM) N-Methylpyrrolidin dazu und rührt über Nacht und unter Stickstoff bei 0°C, wobei das N-Allyl-N-methylpyrrolidiniumsalz der 2-(2-Amino-4-thiazolyl)-2-syn-acetoxyimino-essigsäure auskristallisiert.

d) Anschliessend werden 28ml (248 mM) N-Methylmorpholin und nach 30 Minuten 74 g (220 mM) 2,2-Dithio-bis-benzothiazol dazugegeben. Innert 4 Stunden wird dann unter Rühren eine Lösung von 42 ml (245 mM) Triäthylphosphit in 200 ml Acetonitril bei 0°C dazugetropft. Anschliessend wird noch während 30 Minuten gerührt. Das auskristallisierte Material wird abgenutscht, zuerst mit Acetonitril und dann mit Aether gewaschen und im Vakuum bei 30°C getrocknet. Man erhält den 2-(2-Amino-4-thiazolyl) -2-syn-acetoxyimino-essigsäure-2-benzthiazolyl-thioester vom Smp. 170-172°C.

e) Man suspendiert 29,6 g (80 mM) 7-Amino-3-{[(2,5-dihydro-6-hydroxy-2-methyl-5-oxo-as - 3-triazinyl)thio]methyl}-3-cephem-4-carbonsäure in 560 ml N,N-Dimethylformamid, versetzt mit 24,8 ml (176 mM) Triäthylamin, kühlt auf 0°C ab und gibt 80 ml Wasser dazu. Zur erhaltenen klaren Lösung werden 38 g (100 mM) 2-(2-Amino-4-thiazolyl) - 2-syn-acetoxyimino-essigsäure-2-benzthiazolyl-thioester in fester Form zugegeben, und das Reaktionsgemisch wird 3 Stunden bei 0°C gerührt. Die dunkle Lösung wird filtriert, wobei man zweimal mit je 40 ml N,N-Dimethylformamid wäscht. Man kühlt die Lösung auf 0°C ab, versetzt mit 96 ml (192 mM) einer 2N-Lösung von 2-Aethylcapronsaurem Natrium in Essigester, gibt innert 30 Minuten bei 0°C und unter Rühren 1080 ml Aceton dazu, nutscht das ausgefallene Material ab, wäscht es zuerst mit 500 ml eines Gemisches aus Aceton und N,N-Dimethylformamid im Verhältnis 3:2 und dann mit 500 ml Aceton und trocknet es im Vakuum bei Raumtemperatur. Man erhält das Dinatriumsalz der 7-{2-(2-Amino-4-thiazolyl)-2-syn-acetoxy-acetoxyiminoacetamido] -3-{[(2,5-dihydro-6-hydroxy-2-methyl-5-oxo-as - 3-triazinyl)thio]methyl}-3-cephem-4-carbonsäure, das gemäss HPLC einen Reinheitsgrad von 96,6% aufweist.

f) Man löst 48 g (76,6 mM) des Dinatriumsalzes der 7-[2-(2-Amino-4-thiazolyl)-2-syn-acetoxyiminoacetamido] - 3-{[(2,5-dihydro-6-hydroxy-2-methyl-5-oxo-as - 3-triazinyl)-thio]methyl}-3-cephem-4-carbonsäure in einer auf 25°C abgekühlten Lösung von 520 ml Methanol und 400 ml Wasser. Der pH der Lösung wird mittels 1N-Natronlauge auf 8 gestellt und während der ganzen Reaktionsdauer bei diesem pH gehalten, wobei der Verbrauch an Natronlauge sehr gering ist. Nach beendeter Reaktion (der Reaktionsablauf wird mittels HPLC verfolgt) wird der pH mit 1N-Salzsäure auf 7 gestellt und die dunkel gefärbte Lösung wird während 20 Minuten mit 10 g Aktivkohle gerührt. Anschliessend wird genutscht, wobei man den Rückstand noch mit einem Gemisch aus 52 ml Methanol und 40 ml Wasser wäscht. Die erhaltene gelbe Lösung wird unter Rühren mit 2,4 l Alkohol versetzt, worauf man auf 0°C abkühlt, das ausgefallene Material abnutscht, zuerst mit 350 ml eines Gemisches aus Alkohol und Wasser (6:1) und dann mit 450 ml Alkohol wäscht und anschliessend bei 1600 Pa und Raumtemperatur und dann im Hochvakuum trocknet. Man erhält 34,7 g (77,5%) des Dinatriumsalzes der 7-[2-(2-Amino-4-thiazolyl)-2-syn-hydroxyiminoacetamido] - 3-{[(2,5-dihydro-6-hydroxy-2-methyl-5-oxo-as - 3-triazinyl)thio]methyl}-3-cephem-4-carbonsäure, das noch 5,9% Aethanol und 4,9% Wasser enthält.

Mikroanalyse: [Berechnet für $C_{17}H_{14}N_8Na_2O_7S_3$

(584,51) + 5,9% Aethanol + 4,9% Wasser]:

Berechnet: C 34,24; H 3,48; N 17,10

Gefunden: C 33,79; H 3,20; N 17,24

**Ansprüche**

1. Verbindungen der allgemeinen Formel

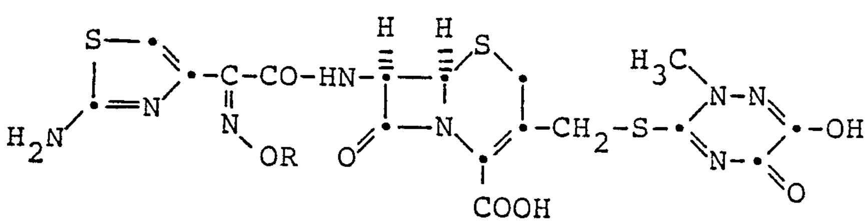

II

worin R niederes Alkanoyl bedeutet,

und Salze davon mit Basen.

2. 7-[2-(2-Amino-4-thiazolyl)-2-syn-acetoxyiminoacetamido] -3-{[(2,5-dihydro-6-hydroxy-2-methyl-5-oxo-as-3-triazinyl)thio]methyl}-3-cephem-4-carbonsäure und Salze davon mit Basen.

3. Verfahren zur Herstellung von Verbindungen gemäss Anspruch 1 oder 2, dadurch gekennzeichnet, dass man die Verbindung der Formel

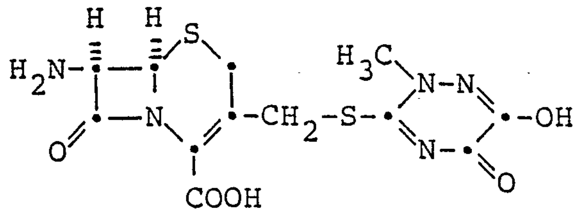

III

mit einer Verbindung der allgemeinen Formel

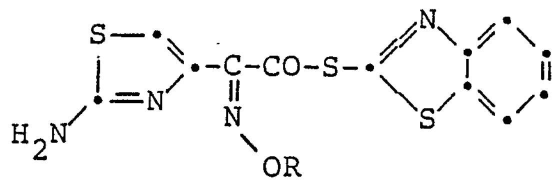

IV

worin R niederes Alkanoyl bedeutet, acyliert und die erhaltene Verbindung der Formel II erwünschtenfalls mit einer Base in ein Salz überführt.

4. Verwendung von Verbindungen gemäss Anspruch 1 oder 2 zur Herstellung der 7-[2-(2-Amino-4-thiazolyl)-2-syn-hydroxyiminoacetamido]-3-{[(2,5-dihydro-6-hydroxy-2-methyl-5-oxo-as-3-triazinyl)thio]methyl}-3-cephem-4-carbonsäure der Formel

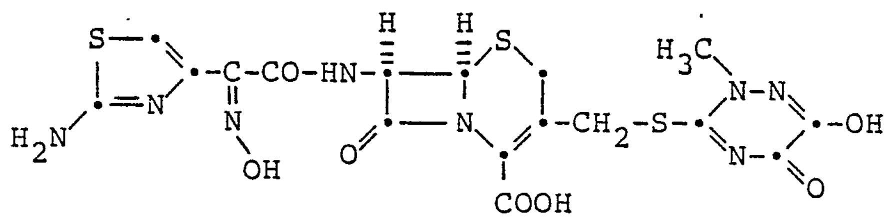

I

und deren pharmazeutisch annehmbaren Salzen.

5. Verfahren zur Herstellung der 7-[2-(2-Amino-4-thiasolyl)-2-syn-hydroxyiminoacetamido]-3-{[(2,5-dihydro-6-hydroxy-2-methyl-5-oxo-as-3-triazinyl)thio]methyl}-3-cephem-4-carbonsäure der Formel

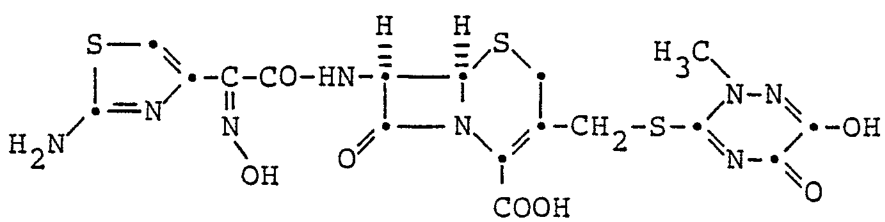

I

und deren pharmazeutisch annehmbaren Salzen, dadurch gekennzeichnet, dass man eine Verbindung gemäss Anspruch 1 oder 2 bei einem pH von 6-9 mit einem Gemisch aus Wasser und einem primären $C_{1-3}$-Alkohol und einer anorganischen Base behandelt und die erhaltene Verbindung der Formel I erwünschtenfalls in ein pharmazeutisch annehmbares Salz überführt.

6. Verfahren nach Anspruch 5, dadurch gekennzeichnet, dass man als primären $C_{1-3}$-Alkohol Methanol verwendet.

7. Verfahren nach Anspruch 5 oder 6, dadurch gekennzeichnet, dass man als anorganische Base Natronlauge verwendet.

8. Verfahren nach einem der Ansprüche 5-7, dadurch gekennzeichnet, dass man die Reaktion bei einem pH von 7,5-8,5 durchführt.

9. Verfahren nach einem der Ansprüche 5-8, dadurch gekennzeichnet, dass das Verhältnis von Wasser zu primärem $C_{1-3}$-Alkohol 1:5 bis 5:1 beträgt.

10. Verfahren nach anspruch 9, dadurch gekennzeichnet, dass das Verhältnis von Wasser zu primärem Alkohol 1:1 bis 1:2 beträgt.

Patentansprüche für den Vertragsstaat AT

1. Verfahren zur Herstellung von Verbindungen der allgemeinen Formel

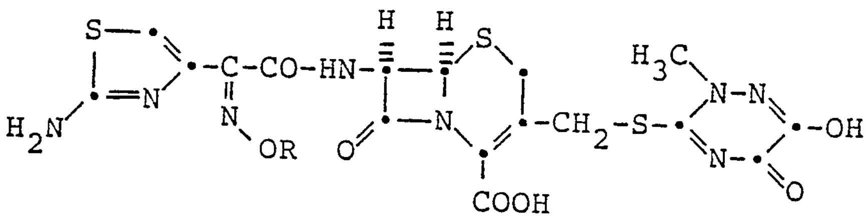

II

worin R niederes Alkanoyl bedeutet,

un Salzen davon mit Basen, dadurch gekennzeichnet, dass man die Verbindung der Formel

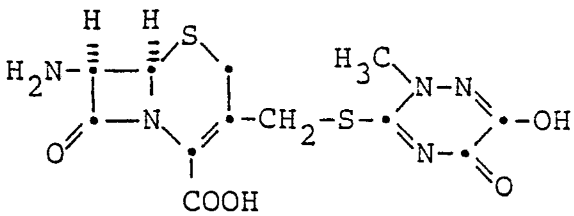

III

mit einer Verbindung der allgemeinen Formel

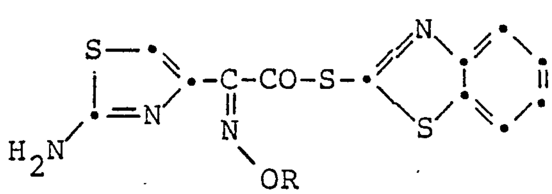

IV

worin R niederes Alkanoyl bedeutet,

acyliert und die erhaltene Verbindung der Formel II erwünschtenfalls mit einer Base in ein Salz überführt.

2. Verfahren gemäss Anspruch 1, dadurch gekennzeichnet, dass man 7-[2-(2-Amino-4-thiazolyl)-2-syn-acetoxyiminoacetamido] - 3-{[(2,5-dihydro-6-hydroxy-2-methyl-5-oxo-as-3-triazinyl)thio]methyl}-3-cephem-4-carbonsäure und Salze davon mit Basen herstellt.

3. Verwendung von Verbindungen der in Anspruch 1 definierten Formel II und Salzen davon mit Basen zur Herstellung der 7-[2-(2-Amino-4-thiazolyl)-2-syn-hydroxyiminoacetamido] - 3-{[(2,5-dihydro-6-hydroxy-2-methyl-5-oxo-as - 3-triazinyl)thio]methyl}-3-cephem-4-carbonsäure der Formel

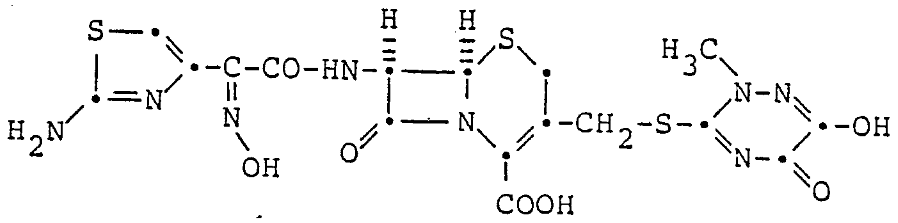

I

und deren pharmazeutisch annehmbaren Salzen.

4. Verfahren zur Herstellung der 7-[2-(2-Amino-4-thiazolyl)-2-syn-hydroxyiminoacetamido]-3-{[(2,5-dihydro-6-hydroxy-2-methyl-5-oxo-as-3-triazinyl)thio]methyl}-3-cephem-4-carbonsäure der Formel

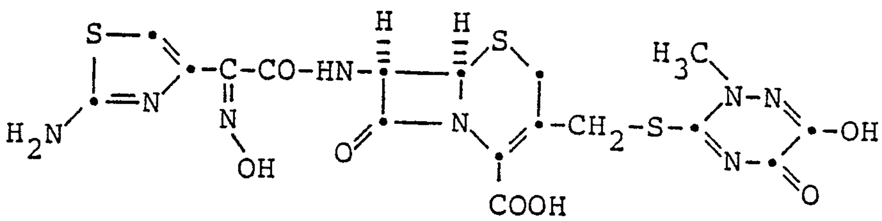

I

und deren pharmazeutisch annehmbaren Salzen, dadurch gekennzeichnet, dass man eine Verbindung der in Anspruch 1 definierten Formel II oder ein Salz davon mit einer Base bei einem pH von 6-9 mit einem Gemisch aus Wasser und einem primären $C_{1-3}$-Alkohol und einer anorganischen Base behandelt und die erhaltene Verbindung der Formel I erwünschtenfalls in ein pharmazeutisch annehmbares Salz überführt.

5. Verfahren nach Anspruch 4, dadurch gekennzeichnet, dass man als primären $C_{1-3}$-Alkohol Methanol verwendet.

6. Verfahren nach Anspruch 4 oder 5, dadurch gekennzeichnet, dass man als anorganische Base Natronlauge verwendet.

7. Verfahren nach einem der Ansprüche 4-6, dadurch gekennzeichnet, dass man die Reaktion bei einem pH von 7,5-8,5 durchführt.

8. Verfahren nach einem der Ansprüche 4-7, dadurch gekennzeichnet, dass das Verhältnis von Wasser zu primärem $C_{1-3}$-Alkohol 1:5 bis 5:1 beträgt.

9. Verfahren nach anspruch 8, dadurch gekennzeichnet, dass das Verhältnis von Wasser zu primärem Alkohol 1:1 bis 1:2 beträgt.